# EUROPEAN PATENT APPLICATION

(11) **EP 2 316 360 A1**
(43) Date of publication of application: **04.05.2011**
(21) Application number: 10182014.0
(22) Date of filing: 13.03.2006
(51) Int. Cl.: A61B 17/34, A61B 17/00, A61M 25/06, A61B 19/00

(54) **Radially expandable access system including trocar seal**

(30) Priority: 16.03.2005 US 81766
(62) Divisional of application: 09006385.0
(71) Applicant: Tyco Healthcare Group LP, Norwalk, CT 06856 (US)
(72) Inventor: Farascioni, David, Bethel, CT, 06801 (US)
(74) Representative: Soames, Candida Jane

(57) **Abstract**

The present disclosure relates to access systems which may be percutaneously or otherwise introduced while in a narrow diameter configuration and, which after introduction, may be radially expanded to accommodate passage of larger diameter surgical instruments therethrough. According to an aspect of the present disclosure, a radially expandable sleeve component (10), for use with an access system, is provided. The sleeve component includes a handle (14) having a passage therethrough; and a sleeve body (12) having a proximal end connected to the handle, a distal end, and an axial lumen aligned with the passage of the handle, the sleeve body having a length. The sleeve body is constructed from a radially expandable braid, wherein the braid is formed of a mesh of non-elastic filaments which axially shortens the length of the sleeve body as the sleeve body is radially expanded. The distal end of the sleeve body is flared radially outward

## Description

### BACKGROUND

### Technical Field

The present disclosure relates generally to apparatus and methods for providing access to an internal operative site during a surgical procedure and, more particularly, to access systems which may be percutaneously or otherwise introduced while in a narrow diameter configuration and which after introduction may be radially expanded to accommodate passage of larger diameter surgical instruments therethrough.

### Background of Related Art

Minimally invasive surgical procedures rely on obtaining percutaneous access to an internal surgical site using small-diameter access tubes (typically 5 to 12 mm), usually referred to as trocars, which penetrate through the skin and which open to the desired surgical site. A viewing scope is then introduced through one such trocar, and the surgeon operates using instruments introduced through other appropriately placed trocars while viewing the operative site on a video monitor connected to the viewing scope. The surgeon is thus able to perform a wide variety of surgical procedures requiring only several 5mm to 12 mm punctures at the surgical site. As a result, patient trauma and recovery time are typically reduced.

Particular minimally invasive surgical procedures are often referred to based on the type of scope used to view the region of the body which is the operative site. For example, procedures in the abdominal area, which rely on a laparoscope for viewing, are typically referred to as laparoscopic procedures. In such laparoscopic procedures, the patient's abdominal region is typically insufflated (filled with pressured gas) to raise the abdominal wall and create sufficient operating space to perform a desired procedure. The trocars used in laparoscopic procedures must therefore include a valve at their proximal end to allow passage of the scope or surgical instruments while inhibiting leakage of the insufflating gas. It has also been proposed to perform laparoscopic procedures by mechanically expanding the abdomen rather than using insufflation.

Recently, a radially expandable access system has been developed, as shown and described in U.S. Pat. Nos. 5,183,464; 5,431,676; 5,814,058; 5,827,319; 6,080,174; 6,245,052; 6,325,812; 6,494,893; and 6,589,225, as well as in U.S. Pat. Appl. Nos. 2001/0039430; 2002/0002360; 2003/0023259; and 2003/0199809, the entire contents of each of which are incorporated herein by reference. The radially expandable access systems disclosed therein may include a pneumoperitoneum needle, an expandable sleeve component which is percutaneously introduced while positioned over the pneumoperitoneum needle, a cannula having a pneumostasis valve permanently affixed at its proximal end, and an obturator which is removably inserted into the cannula to form an expansion member for the sleeve. After the needle/sleeve assembly has been percutaneously introduced, and the peritoneal cavity insufflated in the case of laparoscopic procedures, the needle is removed from the sleeve, and the cannula/obturator assembly introduced through the sleeve. The sleeve, which initially has a diameter in the range of 2-3 mm, is thus expanded to a final diameter depending on the cannula size, which can be selected from 5 mm, 10 mm, or 12 mm. Use of the radially expandable access system has many advantages, including reduced trauma to the patient and the ability to replace a cannula with a larger diameter cannula through a previously introduced sleeve.

While the radially expandable access system represents a substantial advance over conventional trocars, the need and desire exists for improved radially expandable access systems, component kits for such systems, and methods for reconstructing and reusing such systems.

### SUMMARY

The present disclosure relates to access systems which may be percutaneously or otherwise introduced while in a narrow diameter configuration and, which after introduction, may be radially expanded to accommodate passage of larger diameter surgical instruments therethrough.

According to an aspect of the present disclosure, a radially expandable sleeve component, for use with an access system, is provided. The sleeve component includes a handle having a passage therethrough; and a sleeve body having a proximal end connected to the handle, a distal end, and an axial lumen aligned with the passage of the handle, the sleeve body having a length. The sleeve body is constructed from a radially expandable braid, wherein the braid is formed of a mesh of non-elastic filaments which axially shortens the length of the sleeve body as the sleeve body is radially expanded. The distal end of the sleeve body is flared radially outward.

The radially expandable sleeve may further include a sheath substantially encasing the sleeve body. Desirably, the length of the sleeve body is greater than a length of a cannula tube of an expansion assembly when the expansion assembly is operatively associated with the radially expandable sleeve component.

It is contemplated that the flared distal end of the sleeve body facilitates withdrawal of instruments from the radially expandable sleeve component.

According to another aspect of the present disclosure, an access system is provided. The access system includes a radially expandable sleeve component including a handle having a passage therethrough; and a sleeve body having a proximal end connected to the handle, a distal end, and an axial lumen aligned with the passage of the handle, the sleeve body having a length. The distal end of the sleeve body is flared radially outward. The access system further includes a cannula tube having a proximal end, a distal end, and a lumen extending therethrough. The cannula tube is sized for reception in the aperture of the handle of the radially expandable sleeve component. The cannula tube has a length which is shorter than the length of the sleeve body when the cannula tube is fully inserted into the sleeve body of the radially expandable sleeve component.

Desirably, when the cannula tube is fully inserted into the sleeve body of the radially expandable sleeve component the flared distal end of the sleeve body extends beyond the distal end of the cannula tube. The radially expandable sleeve further includes a sheath encasing the sleeve body along at least a portion of the length thereof.

In an embodiment, the sleeve body is constructed from a radially expandable braid. The braid is formed of a mesh of non-elastic filaments which axially shortens the length of the sleeve body as the sleeve body is radially expanded.

Desirably, the sheath maintains the flared distal end of sleeve body in a radially unexpanded condition. It is contemplated that the flared distal end of the sleeve body takes form upon removal of the sheath therefrom.

According to yet another aspect of the present disclosure, an access system is provided. The access system includes a radially expandable sleeve component including a handle having a passage therethrough; and a sleeve body having a proximal end connected to the handle, a distal end, and an axial lumen aligned with the passage of the handle, the sleeve body having a length. The distal end of the sleeve body tapers radially inward. The access system further includes a cannula tube having a proximal end, a distal end, and a lumen extending therethrough. The cannula tube is sized to be received in the aperture of the handle of the radially expandable sleeve component. The cannula tube has a length which is shorter than the length of the sleeve body when the cannula tube is fully inserted into the sleeve body of the radially expandable sleeve component so that the tapered distal end of the sleeve body engages an instrument inserted into the radially expandable sleeve component.

The access system may further include an obturator removably receivable in the lumen of the cannula tube. The obturator has a tapered distal end which extends distally from the distal end of the cannula tube when the obturator is disposed in the lumen of the cannula tube. The access system may further include a pneumoperitoneum needle including a tubular needle; and an internal stylet removably receivable within the tubular needle.

Desirably, when the cannula tube is fully inserted into the sleeve body of the radially expandable sleeve component the flared distal end of the sleeve body extends beyond the distal end of the cannula tube.

The radially expandable sleeve further includes a sheath encasing the sleeve body along at least a portion of the length thereof The sheath desirably maintains the radially inward tapered distal end of the sleeve body in the radially tapered condition. In use, the radially inward tapered distal end of the sleeve body radially expands upon removal of the sheath therefrom.

Other objects and features of the present disclosure will become apparent from consideration of the following description taken in conjunction with the accompanying drawings.

### BRIEF DESCRIPTION OF THE DRAWINGS

By way of example only, embodiments of the radially expandable access system of the present disclosure, will be described with reference to the accompanying drawings, in which:

FIG. 1 is a side view of a radially expandable sleeve component of the access system according to the present disclosure, including a removable sheath encasing a tubular braid portion thereof;

FIG. 1A is a longitudinal cross-secfional view of the radially expandable sleeve component of FIG. 1;

FIG. 2 is a side view of the radially expandable sleeve component of FIG. 1 with the sheath removed from the tubular braid portion thereof;

FIG. 3 is a side view of a prior art pneumoperitoneum needle component for use with the radially expandable sleeve component of FIGS. 1 and 2;

FIG. 4 is a side view of a prior art cannula assembly for use with the radially expandable sleeve component of FIGS. 1 and 2, shown with the cannula body, cannula hub, and valve cap removed or separated from each other, and further shown with the valve cap in partial section;

FIG. 5 is a side view of a prior art obturator component for use with the radially expandable sleeve component of FIGS. 1 and 2, and cannula assembly of FIG. 3;

FIG. 6 is a side view of the radially expandable sleeve component of FIGS. 1 and 2 having the cannula assembly of FIG. 3 operatively associated therewith and with the valve cap of the cannula assembly and the handle of the radially expandable sleeve component shown in partial section;

FIG. 7 is a side view of the radially expandable sleeve component of FIGS. 1 and 2 having the cannula assembly of FIG. 3 operatively associated therewith and a surgical instrument extending therethrough, with the valve cap of the cannula assembly and the handle of the radially expandable sleeve component being shown in partial section;

FIG. 7A is a cross-sectional side view of the radially expandable sleeve component of FIGS. 1 and 2 having the cannula assembly of FIG. 3 operatively associated therewith and a surgical instrument extending therethrough; and

FIGS. 8-13 illustrate use of the radially expandable sleeve component of FIGS. 1 and 2 in providing access to a patient's abdomen.

### DETMLED DESCRIPTION OF REFERRED EMBODIMENTS

The access system of the present disclosure is useful for forming and enlarging percutaneous penetrations into a variety of target locations within a patient's body for a multiplicity of purposes. Such purposes include drainage, intra-organ drug administration, feeding, perfusion, aspiration, and the like, most usually being the introduction of viewing scopes and surgical instruments for use in minimally invasive surgical procedures, such as laparoscopic procedures, thoracoscopic procedures, arthroscopic procedures, endoscopic procedures, and the like. In addition to percutaneous procedures, the access system of the present disclosure will find use in hysteroscopic, colonoscopic, and other procedures where access is established through existing body orifices.

The access systems of the present disclosure are particularly valuable in percutaneous procedures since they will create a very small initial penetration, usually being below about 5 mm, more usually being below about 4 mm, frequently being below about 3.5 mm, and preferably being 3 mm or below. The penetration will be subsequently enlarged to a desired final size, usually having a final diameter in the range from about 5 mm to 15 mm, more usually being from about 5 mm to 12 mm, and typically being from about 5 mm to 10 mm. The enlarged penetration will define an access lumen from the outside of the patient's body to the desired internal location, and it is a particular advantage of the present disclosure that the diameter of the access lumen may be changed as will be described in more detail hereinafter. In non-percutaneous procedures, the access system is valuable since it is capable of passing through the existing body orifice in its narrow-diameter configuration and be subsequently expanded with minimum discomfort and trauma to the patient.

The access system of the present disclosure includes a number of individual components that may be assembled into different size configurations. The assembled components may also be disassembled after use, and the components selectively sterilized or replaced prior to reassembling the access system for further use with a different patient. The different components and component assemblies and subassemblies will be described in greater detail below.

Sterilization of the components of the trocar system disclosed herein may be accomplished by any suitable conventional sterilization technique, including heat, e.g., steam and autoclaving; chemical treatment, e.g., ethylene oxide exposure; radiation, and the like. After use, reusable components will be washed to remove blood and other contaminating substances and then sterilized, preferably by exposure to steam. Disposable components will usually be radiation sterilized in their packages prior to distribution. Thus, disposable components will usually be ready to use out of the package.

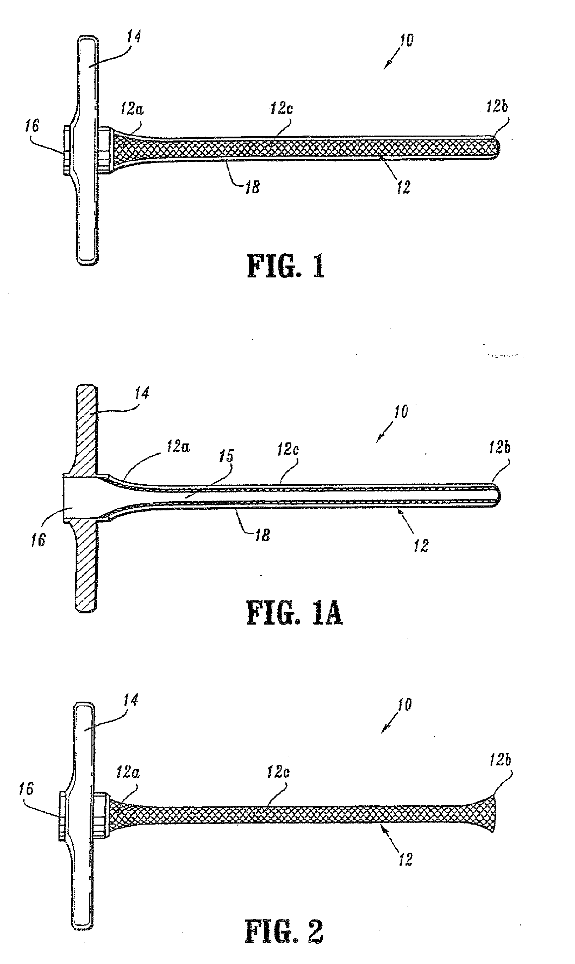

Referring initially to FIGS. 1, 1A and 2, wherein like reference numerals identify similar or identical structural elements, a radially expandable sleeve component or trocar seal, according to an embodiment of the present disclosure, for use as part of an access system, is generally designated as 10. As used herein, the term "distal" refers to that portion of the tool, or component thereof which is further from the user while the term "proximal" refers to that portion of the tool or component thereof which is closer to the user.

As seen in FIGS. 1, 1A and 2, sleeve component 10 includes a sleeve body 12 defining a lumen 15 (see FIG. 1A) from a proximal end 12a to a distal end 12b thereof, and a handle 14 operatively connected to proximal end 12a of sleeve body 12. Preferably, sleeve body 12 is constructed from a radially expandable braid, desirably inelastic, having an inner diameter of about 2 mm and an outer diameter of about 3.5 mm. Handle 14 includes a passage 16 (see FIG. 1A) formed therethrough, which passage 16 is substantially aligned with the lumen of sleeve body 12. Desirably and typically a connector (not shown) is provided about passage 16 for selectively engaging a complementary connector provided on a cannula assembly 40. For example, the complementary connectors may take the form of threads, bayonet fittings, and the like. As will be described in greater detail below, passage of an expansion assembly therethrough causes radial expansion of sleeve body 12, typically to a final diameter of 5 mm, 10 mm, or 12 mm. Radially expandable sleeve 10 may be constructed in accordance with the details set forth in U.S. Pat. No. 5,431,676, the full disclosure of which has been incorporated herein by reference.

As seen in FIG. 2, distal end 12b of sleeve body 12 is flared radially outward. In particular, sleeve body 12 includes an intermediate portion 12c having a uniform diameter along substantially the entire length thereof, and a distal end 12b having a diameter which is larger than the diameter of intermediate portion 12c.

As seen in FIG. 1, a sheath 18 encases and/or otherwise covers sleeve body 12. Sheath 18 extends the entire length of sleeve body 12. Desirably, sheath 18 is fabricated from a plastic or elastomeric material, e.g., polyurethane, tetrafluorethylene, fluorinated ethylene-propylene, or the like. Desirably, sheath 18 will be weakened along an axial line (as by a pair of thin or weakened axial grooves or lines (not shown)) to facilitate splitting of sheath 18 at some point during the procedure. As described in more detail hereinafter, the axial grooves enable sheath 18 to be divided or split along the length thereof, as cannula assembly 40 is received in the lumen of sleeve body 12, and thus allow sleeve body 12 to radially expand.

Additionally, as seen in FIG. 1, sheath 18 helps to maintain flared distal end 12b closed (i.e., in a radially unexpanded condition) prior to introduction of the first surgical instrument. In other words, sheath 18 constricts distal end 12b such that distal end 12b has a diameter which is substantially equal to the diameter of intermediate portion 12c of sleeve body 12.

By way of example only, the braid of sleeve body 12 is preferably formed as a mesh of individual non-elastic filaments (e.g., composed of polyamide fiber, stainless steel, or the like) so that radial expansion causes axial shortening of the braid. Additionally, the braid of sleeve body 12 may be constructed from round filaments, flat or ribbon filaments, square filaments, or the like. Non-round filaments may advantageously reduce the axial force required to provide radial expansion. The filament width or diameter will typically be from about 0.002 inches to about 0.25 inches, usually being from about 0.005 inches to about 0.010 inches.

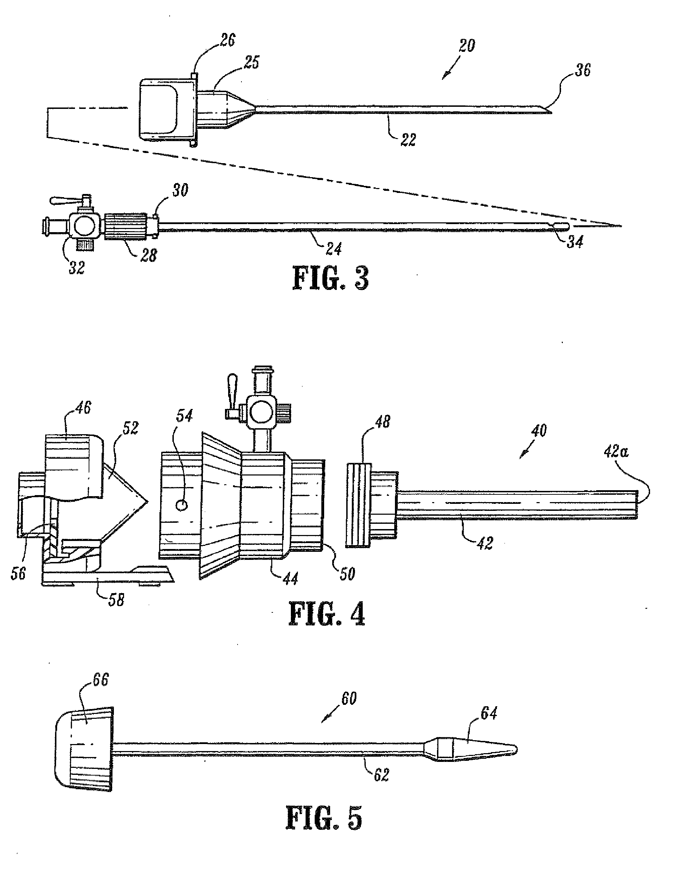

Turning now to FIG. 3, a pneumoperitoneum needle assembly, for use as part of an access system, is generally designated as 20. Pneumoperitoneum needle assembly 20 includes a tubular needle body 22, and a stylet 24 for operative engagement with tubular needle body 22. Tubular needle body 22 includes a hub 25, having a male bayonet connector 26 extending therefrom, provided at a proximal end thereof. Stylet 24 is spring-loaded in a connector 28 which is provided at a proximal end thereof Connector 28 includes a male bayonet fitting 30 which is receivably mounted in a female bayonet fitting (not illustrated) provided in hub 25 of needle body 22. Stylet 24 further includes an insufflation valve 32 provided at a proximal end thereof, and a port 34 formed in a distal end thereof. Accordingly, insufflation gas, introduced through valve 32, is permitted to be released through port 34. In use, stylet 24 is to be mounted within tubular needle body 22 by way of bayonet fittings 30 of connector 28. The distal end of stylet 24 will extend from distal end 36 of needle body 22, and stylet 24 will retract into needle body 22 when needle body 22 is engaged against tissue, as described in more detail below.

Turning now to FIG. 4, a cannula assembly, for use as part of an access system, is generally designated as 40. Cannula assembly 40 includes a cannula tube 42, a cannula hub 44 connectable to cannula tube 42, and a valve cap 46 removably connectable to cannula hub 44. Cannula tube 42 includes a threaded connector 48 at a proximal end thereof which may be removably secured or connected to a fitting 50 provided at a distal end af cannula hub 44. Valve cap 46 desirably includes a pneumostasis valve element 52 and is configured to mate with a male bayonet fitting 54 provided at a proximal end of cannula hub 44. A second disk valve element 56 may be mounted in tandem with the pneumostasis valve element 52 to engage against an outer surface of a surgical instrument (not shown) when the surgical instrument is introduced through cannula assembly 40. Valve element 56 is generally sized for a relatively large instrument, e.g., an instrument having a diameter of about 12 mm. A reducing element 58 may be provided for reducing the size of the port of valve element 56 to accommodate relatively smaller instruments, e.g., instruments having a diameter of about 10 mm.

Turning now to FIG. 5, an obturator, for use as part of an access system, is generally designated as 60. Obturator 60 generally includes a shaft 62, a tapered distal end 64, and a handle 66. Obturator 60 is intended to be placed within a central lumen of cannula assembly 40 in order to form an expansion assembly for use as described below.

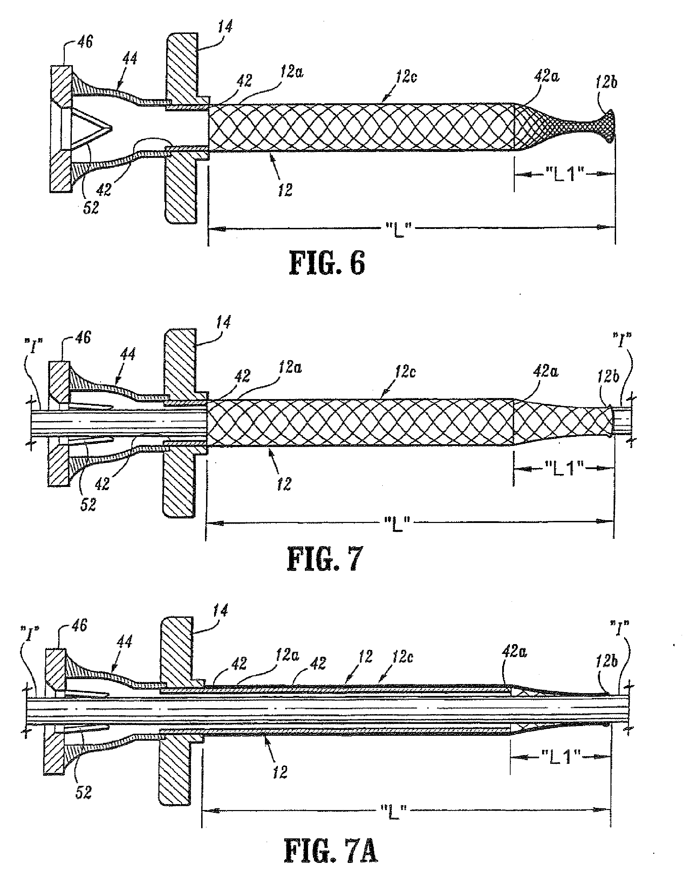

With reference now to FIG. 6, radially expandable sleeve component 10 is shown in operative association with cannula assembly 40. In particular, cannula tube 42 of cannula assembly 40 has been fully inserted into the lumen of sleeve body 12 of expandable sleeve component 10. Desirably, sleeve body 12 has a length "L" which is greater then the length of cannula tube 42 when cannula tube 42 has been fully inserted into expandable sleeve component 10. In this manner, distal end 12b of sleeve body 12 extends distally beyond a distal edge 42a of cannula tube 42. Desirably, length "L" of sleeve body 12 is such that flared distal end 12b thereof is spaced an axial distance "L1" from distal edge 42a of cannula tube 42 when cannula tube 42 is fully inserted into expandable sleeve component 10.

With reference to FIG. 7, flared distal end 12b of sleeve body 12 effectively forms and/or acts as an instrument seal against the surface of an instrument "I" introduced into and extending through cannula tube 42 of cannula assembly 40 and sleeve body 12 of expandable sleeve component 10. Flared distal end 12b of sleeve body 12 is provided in order to facilitate removal of instrument "I" from cannula assembly 40 and, in particular, from sleeve body 12 of expandable sleeve component 10.

While distal end 12b of sleeve body 12 is preferably provided with a flare, it is within the scope of the present disclosure, that distal end 12b of sleeve body 12 does not have to include a flare or the like in order to create and/or act as a instrument seal.

Desirably, as seen in FIGS. 4, 6 and 7, pneumostasis valve element 52 of cannula hub 44 may take the form of a duck bill or "zero" valve. Valve element 52 may include two planar tapering portions which intersect at their distal ends to define an abutment face. The planar tapering portions may each include one or more inwardly directed, longitudinally oriented ribs to facilitate passage of instrument "I". The abutment face permits passage of instrument "I" through valve element 52, but in the absence of instrument "I", and particularly when cannula assembly 40 is inserted into an insufflated body cavity, the abutment face forms a gas-tight seal that isolates the insufflation cavity from the ambient surroundings. Valve element 52 also includes at least one, preferably two, reinforcing ribs (not shown) to stabilize valve element 52. The ribs are positioned to engage instrument "I" to guide instrument "I" through the slit of valve element 52 and prevent piercing of valve element 52 by the tip of instrument "I". Reference may be made to U.S Patent 5,603,702, the entire content of which is incorporated herein by reference, for a more detailed discussion of a valve element.

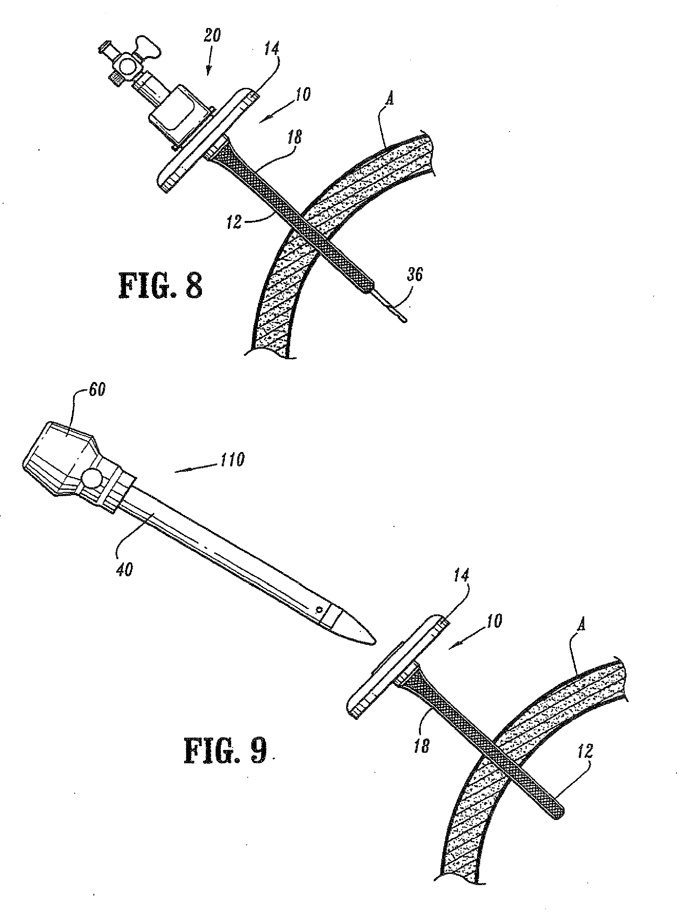

Referring now to FIGS. 8-13, use of radially expandable sleeve component 10, in an access system, will be described in detail. Initially, as seen in FIG. 8, a radially expandable sleeve component 10, having a pneumoperitoneum needle 20 inserted therein, is introduced through a patient's abdomen "A" (or other body location) by engaging sharpened distal end 36 of needle 20 against the tissue and advancing the assembly (e.g., expandable sleeve component 10 operatively coupled with needle 20) until sleeve body 12 of radially expandable sleeve component 10 extends across the tissue.

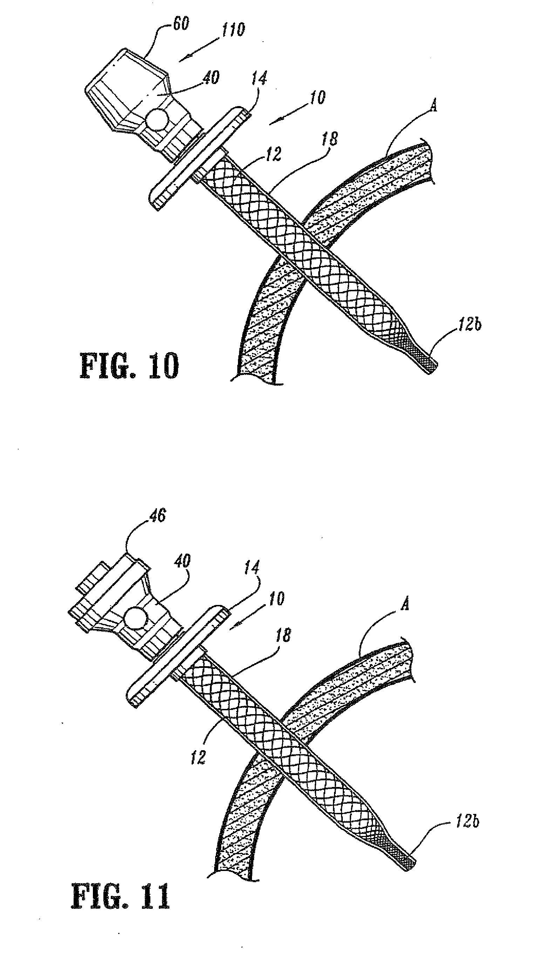

As seen in FIG. 9, needle 20 is removed from expandable sleeve component 10 and an expansion assembly 110, including cannula assembly 40 having obturator 60 operatively associated therewith, is introduced through radially expandable sleeve component 10. Introduction of expansion assembly 110 into radially expandable sleeve component 10 results in radial expansion of sleeve body 12 (see FIG. 10). In so doing, sheath 18 is divided or split along the length of the axial grooves (not shown). Additionally, insertion of expansion sleeve 110 into expandable sleeve component 10 to radially expand sleeve body 12 results in axial shortening of sleeve body 12 to thereby help anchor expansion assembly 110 in place and to help seal the exterior of expansion assembly 110 against the tissue.

As described above, when expansion assembly 110 is fully inserted into radially expandable sleeve component 10, distal edge 42a of cannula tube 42 does not extend beyond distal end 12b of sleeve body 12. Desirably, sleeve body 12 has a length, "L" sufficient that when expansion assembly 110 is fully inserted into sleeve body 12 of expandable sleeve component 10, obturator 60 and cannula tube 40 do not radially expand distal end 12b of sleeve body 12 and, thus, do not split open a distal end of sheath 18.

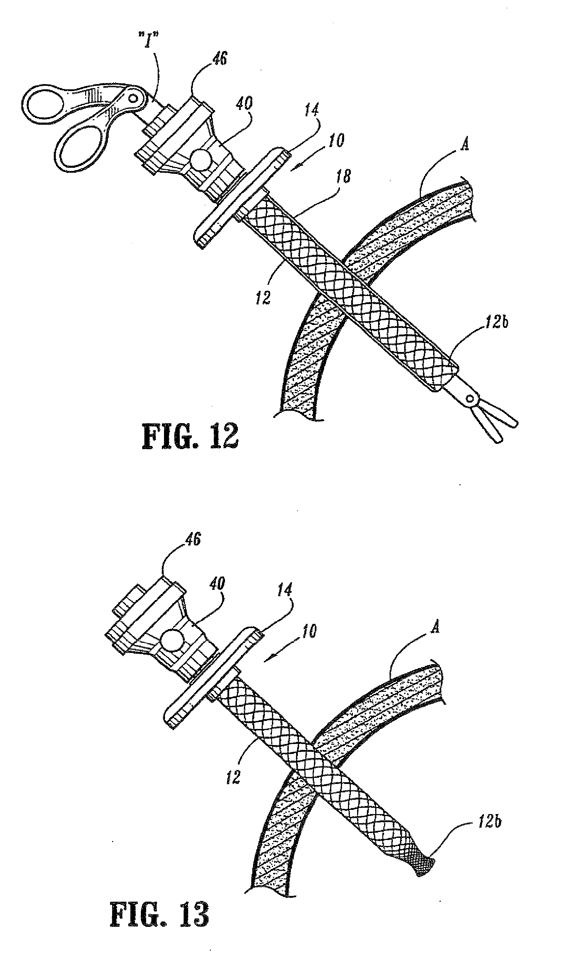

As seen in FIG. 11, obturator 60 may then be removed from cannula assembly 40 and radially expandable sleeve 40, leaving an access channel through abdominal wall "A". With obturator 60 removed, as seen in FIG. 12, a surgical instrument "I" (e.g., surgical graspers, staplers, tackers, fastener appliers, etc.) may be introduced, through cannula assembly 40 and radially expandable sleeve component 10, into the abdominal cavity. Desirably, instrument "I" has a length such that an end effector of instrument "I" is extendable beyond distal edge 42a of cannula tube 42 and beyond distal end 12b of sleeve body 12 of expandable sleeve component 10. Introduction of instrument "I' through distal end 12b of sleeve body 12 results in radial expansion of the same and, thus, in the dividing and/or splitting of the distal end of sheath 18. With sheath 18 divided along its entire length, it is now possible, if desired, to withdraw and remove sheath 18 from between the surface of the incision and sleeve body 12 of expandable sleeve component 10, as seen in FIG. 13.

With reference to FIG. 12, distal end 12b of sleeve body 12 acts as an instrument seal against the outer surface of instrument "I", thereby reducing the escape or passage of insufflation fluid through cannula tube 42. Such a fluid-tight seal is a particular advantage in laparoscopic procedures.

With reference to FIG. 13, following use of surgical instrument "I" in performing the surgical procedure, surgical instrument "I" may be removed and/or withdrawn from expansion assembly 110 and radially expandable sleeve component 10. Flared distal end 12b of sleeve body 12 facilitates the removal and/or withdrawal of surgical instrument "I" from expansion assembly 110 and radially expandable sleeve component 10. Additionally, flared distal end 12b of sleeve body 12 may act like a funnel to facilitate removal and/or retraction of a tissue or organ specimen from the abdominal cavity.

While the above is a complete description of preferred embodiments of the disclosure, various alternatives, modifications, and equivalents may be used. Therefore, the above description should not be taken as limiting the scope of the invention which is defined by the appended claims.

The following numbered paragraphs provide a summary of features of the present disclosure:
1. A radially expandable sleeve component, for use with an access system, the sleeve component comprising:
   a handle having a passage therethrough; and
   a sleeve body having a proximal end connected to the handle, a distal end, and an axial lumen aligned with the passage of the handle, the sleeve body having a length, the sleeve body being constructed from a radially expandable braid, wherein the braid is formed of a mesh of non-elastic filaments which axially shortens the length of the sleeve body as the sleeve body is radially expanded, wherein the distal end of the sleeve body is flared radially outward.
2. The radially expandable sleeve component of 1, further comprising a sheath substantially encasing the sleeve body.
3. The radially expandable sleeve component of 1 or 2, wherein the length of the sleeve body is greater than a length of a cannula tube of an expansion assembly when the expansion assembly is operatively associated with the radially expandable sleeve component.
4. The radially expandable sleeve component of any one of the preceding paragraphs, wherein the flared distal end of the sleeve body facilitates withdrawal of instruments from the radially expandable sleeve component.
5. An access system, comprising:
   a radially expandable sleeve component, including:
   a handle having a passage therethrough; and
   a sleeve body having a proximal end connected to the handle, a distal end, and an axial lumen aligned with the passage of the handle, the sleeve body having a length, wherein the distal end of the sleeve body is flared radially outward; and
   a cannula tube having a proximal end, a distal end, and a lumen extending therethrough, the cannula tube being sized to be received in the aperture of the handle of the radially expandable sleeve component, the cannula tube having a length which is shorter than the length of the sleeve body when the cannula tube is fully inserted into the sleeve body of the radially expandable sleeve component.
6. The access system according to 5, wherein when the cannula tube is fully inserted into the sleeve body of the radially expandable sleeve component the flared distal end of the sleeve body extends beyond the distal end of the cannula tube.
7. The access system according to 5 or 6, wherein the radially expandable sleeve further includes a sheath encasing the sleeve body along at least a portion of the length thereof.
8. The access system according to 7, wherein the sheath maintains the flared distal end of sleeve body in a radially unexpanded condition.
9. The access system according to 5, 6, 7 or 8, wherein the sleeve body is constructed from a radially expandable braid, wherein the braid is formed of a mesh of now-plastic filaments which axially shortens the length of the sleeve body as the sleeve body is radially expanded.
10. The access system according to any one of paragraphs 5 to 9, wherein the flared distal end of the sleeve body takes form upon removal of the sheath therefrom.
11. An access system, comprising:
   a handle having a passage therethrough; and
   a sleeve body having a proximal end connected to the handle, a distal end, and an axial lumen aligned with the passage of the handle, the sleeve body having a length, wherein the distal end of the sleeve body tapers radially inward; and
   a cannula tube having a proximal end, a distal end, and a lumen extending therethrough, the cannula tube being sized to be received in the aperture of the handle of the radially expandable sleeve component, the cannula tube having a length which is shorter than the length of the sleeve body when the cannula tube is fully inserted into the sleeve body of the radially expandable sleeve component so that the tapered distal end of the sleeve body engages an instrument inserted into the radially expandable sleeve component.
12. The access system of 11, further comprising an obturator removably receivable in the lumen of the cannula tube, the obturator having a tapered distal end which extends distally from the distal end of the cannula tube when the obturator is dispose din the lumen of the cannula tube.
13. The access system of 11 or 12, further comprising a pneumoperitoneum needle including:
   a tubular needle; and
   an internal stylet removably receivable within the tubular needle.
14. The access system of 11, 12 or 13, wherein when the cannula tube is fully inserted into the sleeve body of the radially expandable sleeve component the flared distal end of the sleeve body extends beyond the distal end of the cannula tube.
15.The access system of any one of paragraphs 11 to 14, wherein the radially expandable sleeve further includes a sheath encasing the sleeve body along at least a portion of the length thereof.
16. The access system of any one of paragraphs 11 to 15, wherein the sheath maintains the radially inward tapered distal end of the sleeve body in the radially tapered condition.
17. The access system of 16, wherein the radially inward tapered distal end of the sleeve body radially expands upon removal of the sheath therefrom.

## Claims

1. A radially expandable sleeve component, comprising:
a handle having a passage therethrough defining a radial dimension; and
a sleeve body extending distally from the handle along a longitudinal axis, the sleeve body having a length, and being configured and dimensioned to receive a surgical instrument, **characterized in that:**
the sleeve body includes a distal portion with a flared distalmost end, the distal portion defining a first radial dimension smaller than that of the surgical instrument such that the distal portion is expanded radially when the surgical instrument is inserted into the radially expandable sleeve component to a position beyond the flared distalmost end of the sleeve body.

2. The radially expandable sleeve component of claim 1, wherein the sleeve body is constructed from a braid formed of a mesh of non-elastic filaments such that radial expansion of the sleeve body effectuates axial shortening thereof.

3. The radially expandable sleeve component according to any of the preceding claims, wherein the distal portion of the sleeve body is configured, dimensioned, and positioned to engage the surgical instrument in a sealing relationship when the surgical instrument extends beyond the flared distalmost end of the sleeve body.

4. The radially expendable sleeve component according to any of the preceding claims, wherein the flared distalmost end of the sleeve body extends radially outward with respect to the longitudinal axis.

5. The radially expandable sleeve component according to any of the preceding claims, wherein the sleeve body defines a configuration that tapers generally in a distal direction.

6. The radially expandable sleeve component of according to any of the preceding claims, wherein the sleeve body includes a central portion secured to the handle and oriented proximally of the distal portion, the central portion having a length, and defining a second radial dimension that is smaller than that of the surgical instrument such that insertion of the surgical instrument into the central portion of the sleeve body causes radial expansion and axial shortening of the central portion.

7. The radially expandable sleeve component of claim 6, wherein the central portion defines a length, the second radial dimension of the central portion remaining substantially constant along the length of the central portion.

8. The radially expandable sleeve according to claim 7, wherein the length of the sleeve body is sufficient to allow for positioning of the distal portion beneath the tissue, the distal portion of the sleeve body defining a third radial dimension, and the flared distalmost end of the sleeve body defining a fourth radial dimension.

9. The radially expandable sleeve component of claim 8, wherein the third radial dimension of the distal portion is smaller than the fourth radial dimension of the flared distalmost end.

10. The radially expandable sleeve component of claim 9, wherein radial dimension of the passage of the handle is larger than the second radial dimension of the central portion, and the second radial dimension of the central portion is larger than the third radial dimension of the distal portion.

11. The radially expandable sleeve component according to any of the preceding claims, wherein the flared distalmost end of the sleeve body facilitates withdrawal of the surgical instrument from the radially expandable sleeve component.

12. The radially expandable sleeve component according to any one of the preceding claims, further including a sheath encasing the sleeve body along at least a portion of the length thereof.

13. The radially expandable sleeve component according to claim 12, wherein the sheath maintains the flared distalmost end of the sleeve body in a radially unexpanded condition.

14. A surgical access system, comprising:
a radially expandable sleeve component according to any one of the preceding claims; and
a cannula tube having a proximal end, a distal end, and a lumen extending therethrough, the cannula tube being configured and dimensioned for positioning within the passage of the handle of the radially expandable sleeve component.

15. The surgical access system according to claim 14, further comprising an obturator removably receivable in the lumen of the cannula tube, the obturator having a tapered distal end extending distally from the distal end of the cannula tube when the obturator is disposed in the lumen of the cannula tube.
